# DEMANDE DE BREVET EUROPEEN

(11) **EP 1 598 063 A1**
(43) Date de publication de la demande: **23.11.2005**
(21) Numéro de dépôt: 03364025.1
(22) Date de dépôt: 22.10.2003
(51) Int. Cl.: A61K 31/07

(54) **Compositions synergiques de vitamines, de minéraux et d'oligo-éléments pour stimuler l'élimination des lipides intracellulaires**

(71) Demandeur: Shrivastava, Ravi, 63500 Issoire (FR)
(72) Inventeur: Shrivastava, Ravi, 63118 Cebazat (FR); Belani, Hema, 4014 Pune (IN)
(74) Mandataire: Larcher, Dominique

(57) **Abrégé**

Compositions de vitamines, de minéraux et d'oligo-éléments qui stimulent de façon synergique les capacités d'élimination des lipides intracellulaires et qui permettent de diminuer le poids chez l'homme en temps très court sans provoquer d'effets secondaires mais également de prévenir l'effet rebond souvent observé à la fin d'un régime. Ces composition solides ou non solides, utilisables par voie orale ou par voie locale pour diminuer le poids corporel peuvent être facilement fabriquées par l'homme de l'art.
Ces compositions synergiques peuvent être utilisées seules ou également en combination avec des agents qui sont connus pour diminuer la surcharge pondérale, avec un régime alimentaire particulier, avec un appareil d'exercice physique ou un dispositif d'électrostimulation musculaire permettant d'augmenter la dépense énergétique de l'organisme.

## Description

La présente invention concerne l'utilisation d'une composition synergique à base de vitamines, de minéraux et d'oligo-éléments pour stimuler l'élimination des lipides intracellulaires.

Les vitamines, les minéraux et les oligo-éléments sont des micronutriments indispensables au bon fonctionnement métabolique de l'organisme. Les vitamines sont des substances organiques aux structures chimiques variables et parfois complexes, tandis que les minéraux et les oligo-éléments ont une origine minérale.

Ces micronutriments ont chacun un rôle spécifique principal (constitutif, catalytique, coenzyme, prohormone) et aucun d'entre eux ne peut être remplacé par un autre pour assurer le maintien de l'homéostasie.

Les doses actives sont variables. Les vitamines par exemple sont actives à faible dose, les proportions nécessaires pour chacune d'elles variant de 1 à 25 000 si l'on prend les extrêmes que sont respectivement la vitamine B12 pour la plus faible et la vitamine C pour la plus forte. Il en est de même pour les oligo-éléments.

Des valeurs choisies par différents experts tenant compte des données scientifiques concernant les besoins nutritionnels, les motivations et habitudes alimentaires des groupes concernés (enfants, adolescents, adultes, personnes âgées, sportifs...etc.) ont abouti à l'établissement d'Apports Nutritionnels Conseillés (DUPIN H. ABRAHAM J. GIACHETTI I. Apports Nutritionnels conseillés pour la population française, Tec & Doc Lavoisier, Paris, 1999) ou d'établir des Apports Journaliers Recommandés (AJR).

Malgré l'existence de ces recommandations, un nombre grandissant d'études épidémiologiques réalisées dans les pays développés tendent à démontrer l'existence de carences plus ou moins marquées en fonction des micronutriments et du segment de population considéré.

Selon les résultats de trois enquêtes principales menées sur le statut nutritionnel des français, le pourcentage de la population qui ne reçoit pas les apports conseillés pour différentes catégories de micronutriments est le suivant (SOUCCAR T. CURTAY J.P. Le nouveau guide des vitamines, Editions du Seuil, Paris, 1996, page 11) :

| Vitamines, minéraux et oligo-éléments | Pourcentage des hommes ne recevant pas les AJR | Pourcentage des femmes ne recevant pas les AJR | Moyenne |
|---|---|---|---|
| Vitamine A | 11,7 à 60 | 8 à 50 | 32,43 |
| Vitamine B1 | 43 à 80 | 69 à 80 | 68,00 |
| Vitamine B2 | 27 à 60 | 24,6 à 60 | 42,90 |
| Vitamine B3 | 49,5 | 49,3 | 49,40 |
| Vitamine B6 | 67,5 à 80 | 90 à 92 | 82,38 |
| Vitamine B9 | 40 à 90 | 50 à 90 | 67,50 |
| Vitamine C | 25 à 60 | 15 à 60 | 40,00 |
| Vitamine D | 90 à 98 | 90 à 98,6 | 94,15 |
| Vitamine E | 40 à 100 | 75 à 100 | 78,75 |
| Magnésium | 60 | 80 | 70,00 |
| Calcium | 20 | 30,0 | 25,00 |
| Fer | 5 | 55 à 90 | 38,75 |
| Zinc | 80 | 90 | 85,00 |
| | | | Total 59,56 |

Ces études tendent à démontrer que globalement, 6 à 7 personnes sur 10 environ présentent une déficience plus ou moins complète vis à vis d'au moins une vitamine, un minéral ou un oligo-élément mais les conséquences exactes de ces différentes déficiences ne sont pas scientifiquement établies.

Par ailleurs, il n'existe aucun aliment qui contient tous ces éléments vitaux en quantité suffisante et en proportion adéquate.

Le rôle exact et complet des vitamines, des minéraux et des oligo-éléments demeure inconnu. Les proportions respectives entre ces différents éléments vitaux n'ont par ailleurs jamais été scientifiquement établies pour une fonction cellulaire donnée.

A ce jour, aucune corrélation entre les carences en vitamines, en minéraux et en d'oligo-éléments et le problème de surcharge pondérale ou les hypercholestérolémies n'a été scientifiquement établie.

On observe par ailleurs dans les pays développés une augmentation considérable de la proportion d'individus (environ 40% de la population) présentant une surcharge pondérale marquée et de l'excès de poids.

La surcharge pondérale résulte de l'accumulation de grandes quantités de graisses par certaines cellules de l'organisme, les adipocytes. Ces cellules peuvent stocker de grandes quantités de lipides à l'intérieur même de leur cytoplasme, ce sont les lipides intracellulaires. Ce stockage excessif est en réalité lié à la diminution de la capacité d'élimination des déchets et des lipides par les cellules.

De nombreuses stratégies ont été proposées pour diminuer la surcharge pondérale, tel que les régimes alimentaires spécifiques (hypocalorique, diète protéinée...), la consommation d'extraits de plantes pour "brûler les graisses", les médicaments pour diminuer la sensation de faim (isomérides), les traitements hormonaux, la prise en charge médicalisée...etc. Ces traitements provoquent des déséquilibres physiologiques qui peuvent aggraver les déficiences en éléments vitaux, donnent des résultats temporaires, perturbent la physiologie normale des cellules, provoquent des effets néfastes et toxiques ou induisent un effet rebond plus communément désigné sous l'expression de "phénomène de Yo-Yo".

Paradoxalement, alors que le coeur du problème réside dans le dysfonctionnement du métabolisme des adipocytes, aucune de ces stratégies d'action plutôt générale, ne s'est intéressée à la cause même de la surcharge pondérale qui est l'accumulation des lipides au niveau intracellulaire.

Il n'existe pas de traitement tenant compte de la diminution de la capacité des cellules à éliminer les dépôts lipidiques intracellulaires alors que c'est à ce niveau que ce situe la véritable cause de la surcharge pondérale.

La présente invention concerne l'utilisation d'une composition synergique par voie orale ou par voie locale à base de vitamines, de minéraux et d'oligo-éléments pour stimuler de façon ciblée les capacités d'élimination des cellules afin de diminuer l'accumulation des lipides intracellulaires sans induire de toxicité ni d'effets secondaires.

Les produits amincissants pour application locale à base de caféine ont pour effet de mobiliser les réserves de graisse au niveau sous-cutané mais n'agissent pas sur l'accumulation de graisse dans les tissus profonds. Ils n'ont qu'une action superficielle qui ne permet pas de diminuer la surcharge pondérale.

De nombreuses compositions diététiques ou pharmaceutiques par voie orale utilisent des produits naturels ou synthétiques, des extraits de plantes connus pour agir sur la surcharge pondérale. Ces compositions associent par exemple l'effet d'extraits de plantes diurétiques, drainantes, qui gonflent dans l'estomac et le tube digestif pour diminuer la sensation de faim mais ces compositions n'ont pas d'effet spécifique sur l'élimination des lipides intracellulaires. L'utilisation de principes actifs pharmacologiques (caféine, éphédrine, alpha et bêta agonistes) par voie orale dans les compositions diététiques ou alimentaires est interdite ou réglementairement limitée. Les médicaments ont un mode d'action central (coupe-faim) mais ont de nombreux effets secondaires.

Enfin, il existe sur le marché de très nombreuses formulations à base de vitamines, de minéraux et oligo-éléments. Ces produits ont soit une action large et non spécifique, soit sont destinés à une catégorie particulière de la population (enfants, adolescents, adultes, femmes, seniors) mais il n'existe pas de composition synergique à base de vitamines, de minéraux et d'oligo-éléments qui soit destinée à stimuler spécifiquement les capacités d'élimination des lipides intracellulaires afin de diminuer le poids sans induire de toxicité.

Or la demanderesse a découvert avec étonnement que l'excès de poids peut être diminué en un temps très court sans produire d'effets secondaires par l'utilisation de compositions synergiques associant certaines vitamines, certains minéraux et oligo-éléments qui permettent de stimuler l'élimination des réserves lipidiques intracellulaires.

Une composition synergique de vitamines, de minéraux et d'oligo-éléments stimulant fortement et spécifiquement l'élimination des lipides intracellulaires n'a jamais été décrite.

Une augmentation des capacités d'élimination des lipides intracellulaires produit ainsi une diminution du stockage des graisses, de la masse corporelle totale et de la surcharge pondérale. En outre, l'efficacité de ces compositions de vitamines, de minéraux et d'oligo-éléments peut être encore améliorée lorsqu'elles sont combinées avec des extraits de plantes connus pour agir sur la surcharge pondérale par voie orale ou par voie locale ou avec un régime alimentaire particulier.

L'accumulation des lipides intracellulaires a été diminuée *in vitro* par l'utilisation de compositions synergiques associant certaines vitamines, certains minéraux et oligo-éléments, à des doses comprises entre 15 et 200 pour cent des Apports Journaliers Recommandés (AJR), plus particulièrement 50% des AJR.

Les meilleurs résultats ont été obtenus avec des associations spécifiques comprenant 4 vitamines et 4 minéraux ou oligo-éléments : vitamine A, vitamine B2, vitamine B9, vitamine E, magnésium, zinc, cuivre et sélénium (diminution de 89% des lipides intracellulaires).

Les résultats d'une étude clinique de 30 jours réalisée chez des sujets ayant un indice de masse corporelle supérieur de 20% par rapport à la normale a permis de montrer que l'utilisation d'une composition synergique contenant au moins deux vitamines parmi la vitamine A, la vitamine B2, la vitamine B9, la vitamine E, avec au moins deux minéraux ou oligo-élément parmi le magnésium, le zinc, le cuivre ou le sélénium permettait une diminution moyenne du poids corporel de -2,47 kg. L'utilisation de cette composition s'accompagne d'une forte amélioration des paramètres d'appréciation de l'index de bien-être. Les résultats sur le poids sont meilleurs que lors de l'utilisation d'un régime hypocalorique seul. L'efficacité de cette composition synergique de vitamines, de minéraux et d'oligo-éléments est légèrement améliorée lorsqu'elle est utilisée en combinaison avec un complément alimentaire à base de plantes connues pour agir sur la surcharge pondérale ou avec d'autres régimes. Par ailleurs, le poids des volontaires ayant utilisé une composition synergique associant des vitamines, des minéraux et des oligo-éléments est resté stable à la fin des trois mois de la période d'observation ce qui n'a pas été le cas des sujets utilisant un régime hypocalorique seul. Cette observation montre que l'utilisation d'une composition synergique permet en outre de stabiliser le poids afin de prévenir l'effet rebond observé à la fin d'un régime.

C'est pourquoi la présente demande a pour objet l'utilisation de compositions synergiques contenant des vitamines, des minéraux et des oligo-éléments qui permettent de stimuler les capacités d'élimination des lipides intracellulaires pour diminuer la surcharge pondérale en combinaison ou non avec des compositions contenant des plantes connues pour agir sur la surcharge pondérale, avec un régime particulier, un appareil d'exercice physique d'électrostimulation musculaire.

Les vitamines, les minéraux et les oligo-éléments entrant dans la constitution de ces compositions peuvent provenir de différentes origines pour êtres associés sous différentes formes galéniques dont les modes de préparation sont familières à l'homme de l'art.

Dans la présente demande et dans ce qui suit, le terme « composition » selon l'invention vise tant les préparations solides que non solides (liquides, semi-liquides, visqueuses).

Dans des conditions préférentielles de mise en oeuvre de l'invention, les compositions sont des préparations pharmaceutiques ou diététiques pour une utilisation par voie orale.

Dans des conditions préférentielles de mise en oeuvre de l'invention, une telle composition solide peut ainsi se présenter sous forme de poudre lyophilisée ou non, instantanéisée (soupe), sous forme extrudée (granulés, filaments, pâtes), sous forme de sachets, de gélules, de comprimés.

Dans des conditions préférentielles de mise en oeuvre de l'invention, une telle composition non solide peut ainsi se présenter sous forme de solution buvable ou à diluer, de sirop ou d'ampoules monodose.

Dans des conditions préférentielles de mise en oeuvre de l'invention, les compositions sont des préparations pharmaceutiques ou cosmétiques pour une utilisation par voie locale.

Selon un mode de réalisation avantageux, ces formes galéniques sont des compositions pharmaceutiques à libération immédiate ou prolongée permettant une administration par voie locale (crème, gel, système transdermique).

Selon un mode de réalisation avantageux, la composition permettant de stimuler les capacités d'élimination des lipides intracellulaires résulte de l'utilisation d'agents permettant la mise à disposition de l'organisme de vitamines, de minéraux et d'oligo-éléments.

Dans des conditions préférentielles de mise en oeuvre les agents permettant un apport en vitamines pourront être choisis parmi les formes d'apport usuelles synthétiques ou naturelles de vitamines, une source ou un extrait riche en vitamines.

Dans des conditions préférentielles de mise en oeuvre les agents permettant un apport en minéraux et en oligo-éléments pourront être choisis parmi les formes d'apport usuelles synthétiques ou naturelles de minéraux et d'oligo-éléments, une source ou un extrait riche en minéraux ou oligo-éléments, les levures enrichies en minéraux ou oligo-éléments.

De tels agents sont par exemple commercialisés par la société QUIMDIS (France) ou par la société COOPER (Coopération Pharmaceutique Française, France).

La dose usuelle, variable selon la composition choisie peut être par exemple administrée quotidiennement pendant une période au moins égale à 14 jours, 30 jours de préférence, à des doses comprises entre 15 et 200 pour cent des Apports Journaliers Recommandés (AJR) de chacun de ces éléments, plus particulièrement 50% des AJR.

Par AJR, on entend la quantité de micronutriment effectivement disponible pour une utilisation par l'organisme et non pas le poids brut de la forme d'apport utilisée.

Dans des conditions préférentielles de mise en oeuvre, cette composition est utilisée pour accélérer l'élimination des lipides intracellulaires, la détoxication cellulaire afin de diminuer le poids, agir sur l'obésité, le stress, la fatigue, améliorer la forme physique, le sommeil, les troubles liés à l'âge ou le désir sexuel.

Selon le problème visé, ces compositions peuvent être associées avec un médicament, un aliment ou un régime hypocalorique, un produit nutritionnel ou cosmétique pour obtenir des effets synergiques.

C'est pourquoi la présente invention a encore pour objet la combinaison de compositions synergiques pour stimuler les capacités d'élimination des lipides intracellulaires avec des compositions contenant des plantes ou des agents connus pour agir sur la surcharge pondérale.

Dans des conditions préférentielles de mise en oeuvre de l'invention, on utilise différents principes actifs, notamment des extraits de plante.

Par "extrait de plante", l'on entend tant des plantes que des parties de plantes, par exemple et de préférence de plante entière et de préférence séchées ou déshydratées, et broyées, ou encore des extraits de telles plantes ou parties de plantes obtenus à l'aide d'au moins un solvant aqueux et/ou organique et se présentant sous une forme classiquement employée en pharmacie ou diététique, liquide ou solide.

Dans des conditions préférentielles de mise en oeuvre les extraits de plantes et les agents connus pour agir sur la surcharge pondéral être choisis parmi le groupe des extraits de plante contenant naturellement des principes actifs stimulant la thermogenèse, ou contenant des substances cholagogues et cholérétiques, des agents capables de retenir les graisses au niveau intestinal ou de stimuler le transit intestinal.

Ainsi, on peut trouver dans les compositions selon l'invention, une ou plusieurs substances capables de stimuler la thermogenèse, et notamment, de la caféine ou un extrait de plante riche en caféine obtenu par exemple à partir des graines de Guarana (Paullinia cupana), un extrait de Thé vert (Thea sinensis), un extrait de noix de Cola (Cola nitida), des substances cholérétiques et cholagogues, de préférence la cynarine ou un extrait d'Artichaut (Cynara scolymus), certaines plantes épices, de préférence le Gingembre (Zingiber officinalis), des enzymes, de préférence la bromélaïne ou un extrait d'Ananas (Ananas comosus) riche en bromélaïne, la papaïne ou un extrait de Papaye (Carica papaya) riche en papaïne, des fibres végétales, notamment les fibres de fruits (citron, pamplemousse, pomme), de céréales, les pectines, les fructo-oligosaccharides, de préférence l'inuline, le chitosan, l'argile, de préférence une argile verte de type illite à structure phylliteuse, une argile montmorrillonite, kaolinite, attapulgite, sépiolite, smectite et bentonite.

De tels principes actifs ou extraits de plante sont par exemple commercialisés par la société BIOSPHERE (France) ou par la société GREENTECH (France) ou encore ou par la société COOPER (Coopération Pharmaceutique Française, France).

Ces combinaisons peuvent donc se présenter sous forme de poudre lyophilisée ou non, sous forme extrudée (granulés, filaments, pâtes), sous forme de sachets, de gélules, de comprimés.

Ces combinaisons peuvent également se présenter sous forme d'une préparation pharmaceutique ou cosmétique par voie locale, crème, gel, système transdermique.

La présente invention a encore pour objet la combinaison de compositions capables de stimuler les capacités d'élimination des lipides intracellulaires avec un régime alimentaire particulier, un appareil d'exercice physique ou d'électrostimulation musculaire.

Dans des conditions préférentielles de mise en oeuvre les régimes alimentaires particuliers pourront être choisi parmi le groupe des régimes hypocaloriques, des régimes hyperprotéinés, des régimes améliorant le transit intestinal.

Dans des conditions préférentielles de mise en oeuvre, les appareils d'exercice physique ou d'électrostimulation pourront être des appareils ou des dispositifs permettant de faire travailler les muscles afin d'augmenter la dépense énergétique de l'organisme.

Les exemples qui suivent illustrent la présente demande. Ces exemples de compositions pour stimuler les capacités d'élimination des lipides intracellulaires ont été développés sur la base des résultats obtenus au cours des études pharmacologiques. Certaines compositions ont ensuite été soumises à des expérimentations cliniques.

### EXEMPLE 1 :

On a préparé des gélules, des comprimés et des sachets de vitamines, de minéraux et d'oligo-éléments répondant à la formule : (en milligrammes par gélules, par comprimé ou par sachet)

| | |
|---|---|
| Vitamine A (bêta carotène) | 24.0000 |
| Vitamine B2 (riboflavine) | 0.8000 |
| Vitamine B9 (acide folique) | 0.1000 |
| Vitamine E (poudre à 50% de vitamine E) | 10.0000 |
| Magnésium (oxyde de magnésium) | 248.7000 |
| Zinc (oxyde de zinc) | 9.3350 |
| Cuivre (oxyde de cuivre) | 1.2520 |
| Sélénium (sélénate de sodium) | 0.0822 |
| Excipient pour les gélules | qsp 330 mg |
| Excipient pour les comprimés | qsp 400 mg |
| Excipient pour les sachets | qsp 1400 mg |

Posologie: 1 gélule ou 1 comprimé chaque matin avec un verre d'eau ou bien 1 sachet pouvant être mélangé à un yaourt ou à tout autre aliment.

### EXEMPLE 2 :

On a préparé des gélules de vitamines, de minéraux et d'oligo-éléments associés à des extraits de plantes et répondant à la formule :

| | milligrammes / gélule |
|---|---|
| Vitamine A (bêta carotène) | 24.0000 |
| Vitamine B2 (riboflavine) | 0.8000 |
| Vitamine B9 (acide folique) | 0.1000 |
| Vitamine E (poudre à 50% de vitamine E) | 10.0000 |
| Magnésium (oxyde de magnésium) | 248.7000 |
| Zinc (oxyde de zinc) | 9.3350 |
| Cuivre (oxyde de cuivre) | 1.2520 |
| Sélénium (sélénate de sodium) | 0.0822 |
| Extrait de guarana à 25% de caféine naturelle | 25.0000 |
| Extrait d'artichaut à 1% de cynarine | 10.0000 |
| Excipients | qsp 365 mg |

Posologie: 1 gélule chaque matin avec un verre d'eau.

### EXEMPLE 3 :

On a préparé des comprimés de vitamines, de minéraux et d'oligo-éléments associés à des extraits de plantes répondant à la formule :

| | milligrammes / comprimé |
|---|---|
| Vitamine A (bêta carotène) | 24.0000 |
| Vitamine B2 (riboflavine) | 0.8000 |
| Vitamine B9 (acide folique) | 0.1000 |
| Vitamine E (poudre à 50% de vitamine E) | 10.0000 |
| Magnésium (oxyde de magnésium) | 248.7000 |
| Zinc (oxyde de zinc) | 9.3350 |
| Cuivre (oxyde de cuivre) | 1.2520 |
| Sélénium (sélénate de sodium) | 0.0822 |
| Extrait de noix de cola à 25% de caféine | 20.0000 |
| Extrait d'ananas | 10.0000 |
| Fibres de pamplemousse | 10.0000 |
| Excipients | qsp 400 mg |

Posologie: 2 comprimés chaque matin avec un verre d'eau.

### EXEMPLE 4 :

On a préparé des sachets de vitamines, de minéraux et d'oligo-éléments associés à des plantes ou des agents connus pour agir sur la surcharge pondéral répondant à la formule :

| | milligrammes / sachet |
|---|---|
| Vitamine A (bêta carotène) | 24.0000 |
| Vitamine B2 (riboflavine) | 0.8000 |
| Vitamine B9 (acide folique) | 0.1000 |
| Vitamine E (poudre à 50% de vitamine E) | 10.0000 |
| Magnésium (oxyde de magnésium) | 248.7000 |
| Zinc (oxyde de zinc) | 9.3350 |
| Cuivre (oxyde de cuivre) | 1.2520 |
| Sélénium (sélénate de sodium) | 0.0822 |
| Inuline de chicorée | 100.2160 |
| Extrait de guarana à 25% de caféine naturelle | 25.0000 |
| Extrait d'artichaut à 1% de cynarine | 10.0000 |
| Extrait de thé vert à 3% de caféine naturelle | 10.0000 |
| Argile verte | 50.0000 |
| Poudre de jus d'ananas | 10.0000 |
| Tamarin | 7.0000 |
| Gingembre | 7.0000 |
| Gomme Karaya | 6.0000 |
| | |
| Excipients | qsp 1400 mg |

Posologie: 1 sachet par jour avec un verre d'eau ou mélangé avec un yaourt, le matin de préférence.

### EXEMPLE 5 :

On a préparé des gélules de vitamines, de minéraux et d'oligo-éléments répondant à la formule :

| | milligrammes / gélule |
|---|---|
| Vitamine A (bêta carotène) | 8.0000 |
| Vitamine B1 (chlorhydrate de thiamine) | 0.2350 |
| Vitamine B2 (riboflavine) | 0.2670 |
| Vitamine B3 (nicotinamide) | 3.0000 |
| Vitamine B5 (acide pantothénique) | 1.0000 |
| Vitamine B6 (chlorhydrate de pyridoxine) | 0.3350 |
| Vitamine B8 (biotine) | 0.0250 |
| Vitamine B9 (acide folique) | 0.0350 |
| Vitamine B12 (cyanocobalamine) | 0.000165 |
| Vitamine C (acide ascorbique) | 20.0000 |
| Vitamine E *(poudre* à *50%* de *vitamine* E) | 3.3350 |
| Magnésium (oxyde de magnésium) | 83.0000 |
| Calcium (carbonate de calcium) | 333.2000 |
| Fer (gluconate de fer) | 18.6400 |
| Iode (iodure de potassium) | 0.0327 |
| Potassium (chlorure de potassium) | 24.4900 |
| Zinc (oxyde de zinc) | 3.1120 |
| Cuivre (oxyde de cuivre) | 0.4170 |
| Sélénium (sélénate de sodium) | 0.0274 |
| Excipients | qsp 540 mg |

Posologie: 3 gélules par jour avec un verre d'eau, le matin de préférence.

### EXEMPLE 6 :

On a préparé des pâtes contenant une association de vitamines, de minéraux et d'oligo-éléments répondant à la formule :

| | Milligrammes pour 100 grammes de pâtes |
|---|---|
| Vitamine A (bêta carotène) | 24.000 |
| Vitamine B2 (riboflavine) | 0.8000 |
| Vitamine B9 (acide folique) | 0.1000 |
| Vitamine E (poudre à 50% de vitamine E) | 10.0000 |
| Magnésium (oxyde de magnésium) | 248.7000 |
| Zinc (oxyde de zinc) | 9.3350 |
| Cuivre (oxyde de cuivre) | 1.2520 |
| Sélénium (sélénate de sodium) | 0.0822 |
| oeufs frais | 32 000.0000 |
| Semoule de blé dur | qsp 100 grammes |

Posologie: 100 à 200 grammes de pâtes par jour cuite à l'eau.

### EXEMPLE 7 :

Combinaison de la composition synergique de l'exemple 1 avec 10 g dans 15 centilitres d'eau de soupe déshydratée à base de choux, de légumes et de protéines d'origine laitière à consommer à volonté. Cette préparation destinée à un régime hypocalorique appauvri en glucides et en lipides, enrichi en protéines et en fibres alimentaires apporte environ 32 kilocalories pour 10 grammes de soupe déshydratée.

### EXEMPLE 8 :

Combinaison de la composition synergique de l'exemple 1 avec 100 grammes de pâtes enrichies en protéines à consommer midi et soir et des légumes verts à volonté.

### EXEMPLE 9 :

Combinaison de la composition synergique de l'exemple 1 avec des sachets de 1.4 g chacun, à base de plantes connues pour agir sur la surcharge pondérale répondant à la formule:

| | Pourcentage pondéral |
|---|---|
| Inuline de chicorée | 82.555 |
| Soja précuit | 2.500 |
| Germe de blé | 1.675 |
| Extrait d'Artichaut titré à 3% de cynarine | 0.950 |
| Extrait Guarana titré à 10% de caféine | 0.570 |
| Extrait de thé vert titré à 3% de caféine | 0.570 |
| Avoine précuite | 0.250 |
| Fibres de pomme | 0.100 |
| Lithothamne | 0.100 |
| Levures | 0.025 |
| Arôme | 0.095 |
| Excipients | qsp 100% |

Posologie: 1 sachet par jour avec un verre d'eau ou mélangé avec un yaourt, le matin de préférence.

### EXEMPLE 10 :

Combinaison de la composition synergique de l'exemple 1 avec un gel amincissant répondant à la formule :

| | Pourcentage pondéral |
|---|---|
| Caféine | 2.0 |
| Extrait de Centella asiatica | 2.0 |
| Extrait de Mimosa tenuiflora | 1.5 |
| Extrait d'Azadirachta indica | 0.6 |
| Extrait de Garcinia cambogia | 5.5 |
| Excipients | qsp 100% |

Posologie : appliquer une noix de produit matin et soir et faire pénétrer par un léger massage.

### EXEMPLE 11 :

On a préparé un système transdermique de 10 cm² permettant d'apporter chaque jour à l'organisme les quantités de vitamines, des minéraux et d'oligo-éléments indiquées et répondant à la formule :

| | milligrammes / jour |
|---|---|
| Vitamine A (bêta carotène) | 24.000 |
| Vitamine B2 (riboflavine) | 0.8000 |
| Vitamine B9 (acide folique) | 0.1000 |
| Vitamine E (poudre à 50% de vitamine E) | 10.0000 |
| Magnésium (oxyde de magnésium) | 248.7000 |
| Zinc (oxyde de zinc) | 9.3350 |
| Cuivre (oxyde de cuivre) | 1.2520 |
| Sélénium (sélénate de sodium) | 0.0822 |
| Excipients et système adhésif transdermique | qsp 500 mg |

### EXEMPLE 12 :

Combinaison de la composition de l'exemple 11 avec un système transdermique de 10 cm² permettant d'apporter chaque jour à l'organisme les quantités indiquées d'agents connus pour agir sur la surcharge pondérale et des extraits de plantes répondant à la formule :

| | milligrammes / jour |
|---|---|
| Caféine | 10.0 |
| Extrait de Guarana | 20.0 |
| Extrait de Fucus | 10.0 |
| Excipients et système adhésif transdermique | qsp 500 mg |

La présente invention a encore pour objet un procédé de préparation des compositions ci-dessus décrites, caractérisé en ce que l'on mélange, selon des méthodes connues en elles-mêmes, le ou les principes actifs avec des excipients ou solvants acceptables, notamment pharmaceutiquement ou cosmétiquement acceptables ou acceptables dans l'alimentation.

### ETUDES DE TOLERANCE :

Les études de toxicité aiguë chez le rat réalisées selon les lignes directrices de l'OCDE n° 401 sous couvert d'assurance qualité montrent que les préparations utilisées par voie orale présentées en exemple sont non toxiques à la dose de 5 g/kg.

Les études de tolérance locale cutanée et de tolérance oculaire chez le lapin réalisées selon la méthode officielle pour l'appréciation de l'agressivité superficielle cutanée (Arrêté du 11 mai 1993) et la méthode officielle pour l'évaluation de l'irritation oculaire (Arrêté du 9 juin 1992) sous couvert d'assurance qualité montrent que les préparations utilisées par voie locale présentées en exemple sont non irritantes.

### ETUDE PHARMACOLOGIQUE : Recherche d'associations d'agents capables de stimuler les capacités d'élimination cellulaire pour diminuer l'accumulation des lipides intracellulaires.

Pour évaluer l'effet des vitamines, des minéraux et des oligo-éléments sur la capacité des cellules à éliminer les dépôts de lipides intracellulaires, des cultures *in vitro* de cellules musculaires lisses ont été préparées sur des lamelles en plastique de 1 cm de diamètre selon la méthode décrite par Shrivastava et al. (Meth. Find. Exp. Clin. Pharmacol., 15, 345-350, 1993.). Une culture monocouche à 50-60% de confluence a été obtenue en 4 jours. Le sérum hyperlipidémique de lapin a été ajouté au milieu de culture, les cellules ont été incubées à 37°C et 5% de CO2 pendant 48 heures et les dépôts de lipides intracytoplasmiques ont été quantifié. Le milieu de culture a ensuite été remplacé par un milieu blanc prédéfini à 1% de sérum de veau foetal mais ne contenant pas de vitamines, de minéraux et d'oligo-éléments.

Les concentrations désirées de vitamines, de minéraux et d'oligo-éléments ont été préparées dans le milieu blanc prédéfini en dissolvant les substances testées dans le milieu et en remplaçant le milieu de culture original par le milieu contenant les produits à tester.

La cytotoxicité de chaque vitamine, minéral ou oligo-élément a été préalablement déterminée. Aucune des concentrations utilisée n'était cytotoxique. La concentration de produit testé représentait 1/100 des Apports Journaliers Recommandés (AJR) par millilitre de milieu de culture.

Les produits testés étaient les suivants : vitamine A (bêta carotène), vitamine B1 (chlorhydrate de thiamine), vitamine B2 (riboflavine), Vitamine B3 ou PP (nicotinamide), vitamine B5 (acide pantothénique), vitamine B6 (chlorhydrate de pyridoxine), Vitamine B8 ou H, vitamine B9 (acide folique), vitamine B12 (cyanocobalamine), vitamine C (acide ascorbique), vitamine E (acétate de tocophérol), fer (gluconate de fer), iode (iodure de potassium), potassium (chlorure de potassium), zinc (oxyde de zinc), cuivre (oxyde de cuivre), magnésium (oxyde de magnésium), calcium (gluconate de calcium), sélénium (sélénate de sodium). Seuls les éléments ayant montré une certaine activité lors de l'étape de sélection préliminaire ont été retenus pour les expériences suivantes.

Les dépôts de lipides intracellulaires ont été évalués 3 jours après exposition aux produits testés selon la méthode décrite par Shrivastava et al. (Meth. Find. Exp. Clin. Pharmacol., 15, 345-350, 1993.). Les résultats ont été exprimés sous forme de pourcentage de diminution moyen des dépôts de lipides intracellulaires par rapport aux cultures témoin non traitées considérées comme contenant 100% de dépôt lipidiques.

Les résultats suivants ont été obtenus avec les produits testés seuls (tableau 1), avec les associations de deux produits (tableau 2) et avec les associations synergiques de plusieurs micronutriments(tableau 3).

**Tableau 1 :**

| Pourcentage de diminution des dépôts de lipides intracellulaires avec les produits testés seuls (n=24). | | |
|---|---|---|
| Elément | Concentration Testée en µg/ml (0,016% des AJR) | Pourcentage de diminution des lipides intracellulaires |
| Vitamine A | 8,0 | 12,6 ± 2,3 |
| Vitamine B1 | 0,23 | 7,7 ± 1,4 |
| Vitamine B2 | 0,26 | 14,5 ± 3,9 |
| Vitamine B3 | 3,0 | 2,5 ± 1,0 |
| Vitamine B5 | 1,0 | 4,5 ± 2,1 |
| Vitamine B6 | 0,335 | 9,9 ± 2,9 |
| Vitamine B8 | 0,025 | 1,8 ± 0,7 |
| Vitamine B9 | 0,035 | 17,5 ± 4,6 |
| Vitamine B12 | 0,0001 | 8,2 ± 2,5 |
| Vitamine C | 20,0 | 11,2 ± 4,1 |
| Vitamine E | 3,33 | 21,4 ± 3,9 |
| Magnésium | 66,67 | 22,0 ± 5,2 |
| Calcium | 178,0 | 0 |
| Fer | 18,64 | 6,3 ±2,7 |
| Iode | 0,03 | 0 |
| Potassium | 24,49 | 0 |
| Zinc | 3,11 | 16,3 ± 4,4 |
| Cuivre | 0,42 | 24,3 ± 6,2 |
| Sélénium | 0,013 | 24,4 ± 5,3 |

Ces résultats montrent que les produits testés seuls n'ont pas d'effet sur la diminution de l'accumulation des déchets lipidiques intracellulaires sauf pour la vitamine A, la vitamine B2, la vitamine B9, la vitamine E, le magnésium, le zinc, le cuivre et le sélénium.

**Tableau 2 :**

| Pourcentage de diminution des dépôts de lipides intracellulaires avec les associations de deux produits (n=24) | |
|---|---|
| | Pourcentage de diminution des lipides intracellulaires |
| Vitamine A + vitamine B2 | 20,2 ± 4,6 |
| Vitamine A + vitamine B9 | 26,3 ± 5,5 |
| Vitamine A + vitamine E | 29,2 ± 6,7 |
| Vitamine A + magnésium | 26,1 ± 4,4 |
| Vitamine A + zinc | 18,3 ± 4,4 |
| Vitamine A + cuivre | 21,3 ± 9,1 |
| Vitamine A + sélénium | 17,2 ± 6,1 |
| Vitamine B2 + vitamine B9 | 18,3 ± 2,7 |
| Vitamine B2 + vitamine E | 20,0 ± 4,1 |
| Vitamine B2 + magnésium | 30,2 ± 8,1 |
| Vitamine B2 + zinc | 21,1 ± 6,6 |
| Vitamine B2 + cuivre | 23,3 ± 7,3 |
| Vitamine B9 + vitamine E | 24,4 ± 6,6 |
| Vitamine B9 + magnésium | 18,1 ± 8,2 |
| Vitamine B9 + zinc | 18,4 ± 3,2 |
| Vitamine B9 + cuivre | 22,6 ± 4,9 |
| Vitamine E + magnésium | 25,9 ± 4,9 |
| Vitamine E + zinc | 26,6 ± 7,6 |
| Vitamine E + cuivre | 17,7 ± 2,7 |
| Zinc + cuivre | 16,6 ± 4,9 |
| Zinc + magnésium | 21,9 ± 7,2 |
| Cuivre + magnésium | 24,6 ± 6,2 |
| Vitamine B2 + sélénium | 24,6 ± 2,7 |
| Vitamine E + sélénium | 22,2 ± 7,8 |
| Magnésium + sélénium | 23,7 ± 8,2 |

Ces résultats montrent que l'association de deux vitamines, minéraux ou oligo-éléments n'améliore pas l'élimination des lipides intracellulaires. L'activité n'est pas additive. L'activité de l'association de magnésium et de vitamine B2 est supérieure à celle des deux éléments pris séparément.

**Tableau 3 :**

| Pourcentage de diminution des dépôts de lipides intracellulaires avec les associations synergiques de plusieurs micronutriments (n=24) éventuellement en combinaison avec des extraits de plantes. (vitamine A = A, vitamine B2 = B2 et ainsi de suite pour les vitamines ; les symboles internationaux de la table périodique des éléments désigne les minéraux et les oligo-élément correspondants). | |
|---|---|
| | Pourcentage de diminution des lipides intracellulaires |
| A + B2 + B9 + E | 26.4 ± 6.9 |
| A + B2 + Mg + Zn | 70.0 ± 9.3 |
| A + B5 + B6 + B8 | 22.4 ± 7.6 |
| A + Fe + I + K | 15.3 ± 4.3 |
| B9 + Fe+ Mg + Zn | 31.2 ± 8.4 |
| A + B2+ Cu + Se | 68.4 ± 8.8 |
| A + B2 + Mg + Se | 81.2 ± 11.4 |
| B5 + B6 + Cu + Se | cytotoxique |
| B2 + B9 + Mg + Zn | 68.8 ± 11.4 |
| B2+ B9 + Zn + Se | 77.3 ± 9.9 |
| B2 + B9 + Mg + Se | 76.3 ± 13.2 |
| B2 + B9 + Cu + Se | 80.4 ± 8.2 |
| B2 + Mg + Cu + Se | 34.9 ± 6.7 |
| B5 + B6 + B8 + Fe + I | 24.6 ± 7.2 |
| A + B2 + Mg + Cu | 76.3 ± 14 . 6 |
| B2 + B9 + E + Zn | 42.6 ± 14.6 |
| D + E+ Zn + Si | 52.0 ± 9.0 |
| E + Ca + Fe + Cr | 36.4 ± 8.8 |
| A + B2 + B9 + E + Mg + Zn + Cu + Se | 89.2 ± 12.6 |
| A + B2 + B9 + Mg + Zn + Cu + Se | 72.1 ± 11.6 |
| B2 + B9 + E + Mg + Zn + Cu +Se | 81.6 ± 12.0 |
| A + E + Mg + Zn + Cu + Se | 71.6 ± 9.9 |
| A + E + Zn + Cu + Se | 61.4 ± 13.0 |
| E + Mg + Zn + Cu + Se | 40.1 ± 8.8 |
| A + B2 + Mg + Cu + caféine (0.01%) | 84.4 ± 14.6 |
| A + B2 + Mg + Cu | |
| + Phytoestrogènes de soja (10%) | 83.4 ± 17.3 |

Les résultats montrent que pour éliminer les lipides intracellulaires, il est indispensable d'associer au moins deux vitamines parmi la vitamine A, la vitamine B2, la vitamine B9 et au moins deux minéraux ou oligo-éléments parmi le magnésium, le zinc, le cuivre et le sélénium. Les meilleurs résultats sont obtenus en associant ces 8 éléments. L'activité diminue en l'absence de l'un de ces éléments.

### ETUDE CLINIQUE : Confirmation des résultats chez l'homme

Des gélules, des comprimés et des sachets contenant 50% des AJR en vitamine A, B2, B9, E, en magnésium, zinc, cuivre et sélénium, ont été préparés (1 gélule ou 1 comprimé ou 1 sachet par jour selon la forme utilisée).

Des volontaires adultes de sexe masculin (environ 35%) ou féminin (environ 65%) ayant un indice de masse corporelle de 20% supérieur a la normale ont été sélectionnés pour évaluer l'effet de différents régimes sur l'évolution du poids corporel sur une période de 30 jours. Le poids des volontaires a également été évalué 3 mois après la date de début de l'étude.

La diminution du poids corporel était liée à l'amélioration des fonctions métaboliques cellulaires qui facilite l'élimination des lipides intracellulaires et des déchets de l'organisme. L'effet sur différents paramètres de bien être a été évalué en utilisant des échelles de scores allant de 0 à 4. Cinq groupes étudiés dans des conditions identiques ont été constitués pour l'étude conduite par un Institut de recherche accrédité par le Ministère de la Recherche.

Groupe 1 : complément alimentaire de vitamines, de minéraux et d'oligo-éléments selon l'exemple 1.

Groupe 2 : combinaison d'un complément alimentaire de vitamines, de minéraux et d'oligo-éléments associé à un complément alimentaire à base de plantes selon l'exemple 9.

Groupe 3 : combinaison d'un complément alimentaire de vitamines, de minéraux et d'oligo-éléments associé à un régime contenant des légumes verts à volonté, des fruits et 100 grammes de pâtes hyperprotéinées à consommer midi et soir selon l'exemple 8.

Groupe 4 : régime à base de soupe de légumes lyophilisée sous forme de préparation instantanée à mélanger avec de l'eau bouillante.

Groupe 5 : combinaison d'un complément alimentaire de vitamines, de minéraux et d'oligo-éléments associé à régime à base de soupe de légumes lyophilisée sous forme de préparation instantanée à mélanger avec de l'eau bouillante selon l'exemple 7.

Les résultats moyens suivants ont été obtenus :

| | Nombre de volontaires | | Perte de poids moyenne en 30 jours | | | Index de bien-être (1 à 4) |
|---|---|---|---|---|---|---|
| | H | F | H | F | Moyenne | |
| Groupe 1: | 8 | 30 | -2.22 | -2.61 | -2.47 | 3.21 |
| Groupe 2: | 9 | 18 | -3.67 | -3.82 | -3.76 | 3.42 |
| Groupe 3: | 4 | 12 | -2.10 | -2.93 | -2.62 | 3.11 |
| Groupe 4: | 7 | 14 | -1.15 | -1.30 | -1.22 | 1.80 |
| Groupe 5: | 12 | 18 | -2.20 | -2.75 | -2.56 | 2.89 |

Ces résultats montrent que l'association de 50% des AJR en vitamine A, vitamine B2, vitamine B9, vitamine E, magnésium, zinc, cuivre et sélénium, diminue le poids chez les hommes et chez les femmes (moyenne de -2.47 kg en 30 jours) en accélérant l'élimination des lipides intracellulaires. Une amélioration des résultats est obtenue lorsque la composition synergique est combinée avec un complément alimentaire à base de plantes. Ces résultats peuvent également êtres améliorés en combinaison avec d'autres régimes mais la diminution de poids supplémentaire obtenue n'est pas toujours significative ce qui montre que la plus grande part de la perte de poids observée est due à la composition synergique de vitamines (A, B2, B9, E), de minéraux et d'oligo-éléments (magnésium, zinc, cuivre, sélénium).

Les volontaires du groupe 4 recevant seulement un régime à base de soupe de légume ont montré une diminution de poids très inférieure aux groupes recevant la composition synergique de vitamines, de minéraux et d'oligo-éléments.

Par ailleurs, le poids des volontaires ayant utilisé une composition synergique associant des vitamines, des minéraux et des oligo-éléments est resté stable à la fin des trois mois de la période d'observation ce qui n'a pas été le cas des sujets utilisant un régime hypocalorique seul. Cette observation montre que l'utilisation d'une composition synergique permet en outre de stabiliser le poids afin de prévenir l'effet rebond observé à la fin d'un régime.

Chez les sujets étudiés, l'index de bien-être a été fortement augmenté avec la composition synergique de vitamines, de minéraux et d'oligo-éléments qui améliore remarquablement les paramètres d'évaluation du stress, du sommeil, de la forme physique et de la fatigue ce qui montre que la composition synergique de vitamine A, vitamine B2, vitamine B9, vitamine E, magnésium, zinc, cuivre et sélénium exerce une action favorable sur la santé en général.

## Revendications

1. Utilisation d'une composition synergique par voie orale pour diminuer l'accumulation des lipides intracellulaires **caractérisée en ce qu'**elle comprend au moins deux vitamines choisies parmi la vitamine A, la vitamine B2, la vitamine B9, la vitamine E avec au moins deux minéraux ou oligo-éléments choisis parmi le magnésium, le zinc, le cuivre et le sélénium pour traiter la surcharge pondérale.

2. Utilisation d'une composition synergique par voie orale pour diminuer l'accumulation des lipides intracellulaires **caractérisée en ce qu'**elle comprend au moins deux vitamines avec deux minéraux ou oligo-éléments pour traiter la surcharge pondérale.

3. Utilisation d'une composition synergique selon les revendications 1 à 2 **caractérisée en ce qu'**elle est utilisée par voie locale.

4. Utilisation d'une composition synergique selon les revendications 1 à 3 en combinaison avec une composition par voie orale à base de d'agents qui stimulent la thermogenèse, ont un effet cholagogue ou cholérétique ou qui sont connus pour diminuer la surcharge pondérale.

5. Utilisation d'une composition synergique selon les revendications 1 à 3 en combinaison avec un régime alimentaire hypocalorique, hyperprotéiné ou stimulant le transit intestinal.

6. Utilisation d'une composition synergique selon les revendications 1 à 3 en combinaison avec un appareil d'exercice physique ou un dispositif d'électrostimulation musculaire.

7. Une combinaison de compositions telles que définies à l'une des revendications 1 à 6 à titre de produit pharmaceutique, cosmétique, diététique ou nutritionnel.

8. Une combinaison de compositions telles que définies à l'une des revendications 1 à 6 et un excipient pharmaceutiquement ou cosmétiquement acceptable.

9. Une combinaison de compositions telles que définies à l'une des revendications 1 à 6 et un excipient acceptable dans l'alimentation.
